Europäisches Patentamt

European Patent Office

Office européen des brevets

(11) Publication number: **0 393 818**
**A1**

## EUROPEAN PATENT APPLICATION

(21) Application number: **90301828.1**

(22) Date of filing: **20.02.90**

(51) Int. Cl.⁵: **C12P 7/56**

(30) Priority: **18.04.89 US 340105**

(43) Date of publication of application:
**24.10.90 Bulletin 90/43**

(84) Designated Contracting States:
**AT DE FR GB IT NL SE**

(71) Applicant: **MICHIGAN BIOTECHNOLOGY INSTITUTE**
**3900 Collins Road**
**Lansing Michigan 48910(US)**

(72) Inventor: **Glassner, David A.**
**445 Satinwood Road**
**Okemos, Michigan 48864(US)**
Inventor: **Datta, Rathin**
**442 W. Melrose Avenue, Nr. 3**
**Chicago, Illinois 60657(US)**

(74) Representative: **Newell, William Joseph et al**
**Wynne-Jones, Lainé & James 22 Rodney Road**
**Cheltenham Gloucestershire GL50 1JJ(GB)**

(54) Process for production and purification of lactic acid.

(57) A process for producing and purifying lactic acid comprises growing a lactate salt-producing microorganism on an inexpensive substrate containing carbohydrate, corn steep liquor and corn oil until most of the carbohydrate is converted to lactate. The fermentation broth is then electrodialyzed to recover and concentrate the lactate salt in an aqueous stream which is subjected to water-splitting electrodialysis to form base and a lactic acid product. The resulting lactic acid product is treated first with a strongly acidic ion exchanger in the acid form to remove any sodium or other cations and then treated with a weakly basic ion exchanger in the free base form to remove any sulfate ions or sulfuric acid and to obtain a highly purified lactic acid product.

EP 0 393 818 A1

## PROCESS FOR PRODUCTION AND PURIFICATION OF LACTIC ACID

The present invention relates to an improved process for the production and purification of lactic acid by fermentation, electrodialysis and ion exchange.

Lactic acid is a specialty chemical used in the food and chemical industries. Lactic acid is manufactured by synthetic and fermentative methods for use in food manufacturing, metal pickling, leather tanning, and pharmaceuticals. Lactic acid is a potential commodity chemical because it can be used to make biodegradable polymers or hydrogenated to make propylene glycol and other three carbon chemical intermediates. Lactate salts can be produced by fermentation of many inexpensive carbohydrate substrates. Currently, fermentation lactic acid is expensive to produce because the fermentation process generates a crude lactate salt from which the cells and protein have to be removed.

Lactic acid is currently produced by synthetic and fermentative methods. It can be produced by hydrolyzing lactonitrite. Lactonitrile is manufactured from equimolar amounts of acetaldehyde and hydrogen cyanide. The fermentative routes use Lactobacillus sp. to produce a calcium lactate salt.

In order to develop a commercially attractive process to produce lactic acid by fermentation, several important fermentation and product purification criteria need to be accomplished. The fermentation should be high yield (wt %) and produce a high product concentration using inexpensive substrates and nutrients. Since anaerobic fermentations are run at neutral or near neutral pHs, salts of organic acids rather than the acids themselves are produced. The fermentation broth also contains cells, protein and other undesirable materials. The desired form from the process is purified lactic acid which can be used for specialty chemical or commodity chemical manufacture.

It is the primary object of the present invention to disclose an improved, economically attractive, lactic acid production and purification process.

We have now discovered an improved process for the production of lactic acid which includes a fermentation process which uses a robust strain of a lactate-producing microorganism which produces lactate salt in high concentration from a low cost fermentation medium and a purification process which uses conventional electrodialysis to recover and concentrate the lactate from a whole broth containing cells and nitrogenous impurities; a water-splitting electrodialysis to convert the lactate obtained to lactic acid and base; and, treatment with ion exchangers to remove charged impurities from the lactic acid.

As a result of our discovery, we are able to produce a lactic acid product containing less that 1% nitrogenous material (protein) and less than 10 ppm of contaminating sulfate ions.

In the drawings:

Fig. 1 is a flow diagram showing the process of the present invention; and

Fig. 2 is a schematic illustration of the preferred electrodialysis system.

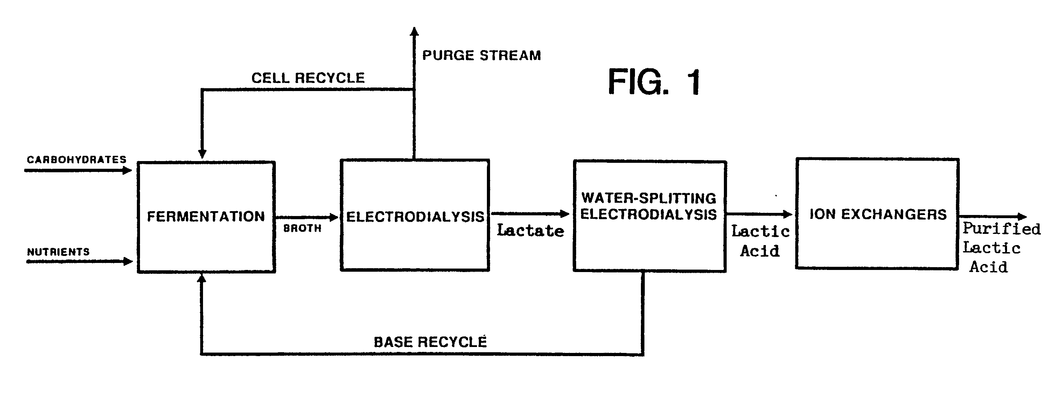

A schematic process flow diagram for the lactic acid production and purification processes of the present invention is shown in Figure 1. The first step is the high yield, high productivity fermentation using the low cost nutrients corn steep liquor and corn oil. Carbohydrates and nutrients are converted to lactate salts in the fermentor. The whole broth is then continuously fed to the conventional electrodialyzer. From the electrodialyzer a lactate depleted cell containing broth is recycled to the fermentor. A small fraction is purged and the cells are removed from this system. The purified and concentrated (2.5 fold) lactate salts from the conventional electrodialysis processing are fed to an evaporator. The concentrated salt (approximately 2 to 2.5 fold over the entering stream) is then fed to the water-splitting electrodialyzer. The water-splitting electrodialysis unit produces a nearly cation free lactic acid stream (85-99 + % cations removed) and an alkali stream for recycle to the fermentor. The lactic acid stream containing residual cations and anions, is sent through two ion exchange polishing columns. Lactic acid with a purity greater than 99% and a concentration of approximately 50% by weight is the product from the process.

In the preferred practice of the present invention, a substantially pure culture of Lactobacillus acidophilus (ATCC 53861) is grown at a controlled pH between about 4.8 to about 5.7 in a fermentor on a medium containing carbohydrates preferably dextrose; about 2% corn steep liquor (dry basis); and corn oil until a yield of about 80 weight percent of lactate salt based on the weight of the carbohydrate is obtained and the fermentation broth contains at least about 20 g/l of lactate. The fermentation is usually complete in about 50 to about 80 hours.

The carbohydrate used in the practice of this invention can be any carbohydrate that is fermented by the strain of bacterium used. For L. acidophilus, these carbohydrate sources include dextrose, sucrose, fructose, lactose, soluble starches, and corn syrups. The fermentation is conducted in an aqueous medium also containing crude corn oil, preferably 0.1% to 1% by weight. The preferred medium also contains about 1% to 4% corn steep liquor on a dry basis to provide the nutrients and growth factors needed for the growth and the reproduction of the microorganism.

The concentration of carbohydrate in the medium is between about 20 g/l to about 120 g/l, preferably between about 40 g/l and about 100 g/l. Carbohydrate concentrations above about 120 g/l give solutions with such high osmotic pressures that the organisms do not grow well. Although the organisms will grow in solutions containing less than 20 g carbohydrate per liter, the concentration of product is so low that its recovery usually is not practical.

In order to obtain good production of lactate salt, the pH of the medium is maintained in the range of from about 4.8 to about 5.7. The pH is conveniently maintained by the addition of alkaline carbonates, alkaline earth hydroxides, or mixtures thereof. Especially preferred are sodium hydroxide and ammonium hydroxide.

The fermentation process of this invention is carried out with agitation at a temperature between about 20°C and about 50°C. Optimum growth of the L. acidophilus organism is at about 39°C. Generally, a 5% inoculum is employed and the medium is first sterilized by heat or other means well known in the fermentation art.

The lactate salt-containing whole broth, including cells, is transported from the fermentor and subjected to electrodialysis to recover and concentrate the lactate salt in an aqueous stream. The viable cells are recycled back to the fermentor. The lactate salt-containing aqueous stream is subjected to water-splitting electrodialysis to form an aqueous lactic acid solution and a base which can be recycled to the fermentor. The aqueous lactic acid solution is then subjected to an ion exchange polish purification with first a strongly acid cationic exchanger and then a weakly basic anionic exchanger to remove positively charged and negatively charged impurities and to yield a highly purified form of lactic acid. The final product, which is an aqueous solution, preferably will contain lactic acid of about 90 to about 100% purity, less than 1% nitrogenous impurities and less then 10 ppm of sulfate ions or other contaminating ions.

The preferred microorganism, L. acidophilus ATCC 53861, has been demonstrated to grow well on media containing carbohydrates, preferably dextrose; corn steep liquor; and at about 0.1 to 1% crude corn oil at a temperature of about 39°C. This strain is capable of producing high concentrations of lactate (75-90 g/l) at cell concentrations of about $2.5 \times 10^{10}$ to about $3.0 \times 10^{10}$ cells per ml. with a high productivity (2.0 to 2.5 g/l hr). It also can produce a high concentration of lactate salt relative to cell population so that electrodialysis using special ion exchange membranes can be successfully used on whole cell-containing broth, as well as cell-free clarified broth, to purify and concentrate the lactate salt into a purified salt product. The performance of the separation process on whole broth is identical to that on clarified broth. Surprisingly, the cells in the whole broth do not foul the electrodialysis membranes. This allows the cell-containing broths to be sent directly to the electrodialysis system without pretreatment such as filtration or centrifugation.

Following the purification and recovery of the lactate salt by electrodialysis, the removal of the sodium ions or other suitable cations can be accomplished using water-splitting electrodialysis. Energy efficient bipolar membranes are used to remove most of the salt cation yielding an acid stream and a base stream for recycle to the fermentor.

The acid stream contains sodium cation and sulfate and other anions which can be removed from the process stream using polish ion exchange columns. Treatment of the acid stream first with strongly acidic ion exchanger and then with a weakly basic ion exchanger (Dowex 50Wx8 or Rohm and Haas IR-120 plus and Rohm and Haas IRA-94, respectively) are used to remove sodium and other cations, and then sulfate and other anions, respectively. This processing also removes some residual contaminating amino acids. The resulting product is a purified aqueous lactic acid solution suitable for use to produce specialty and commodity chemicals.

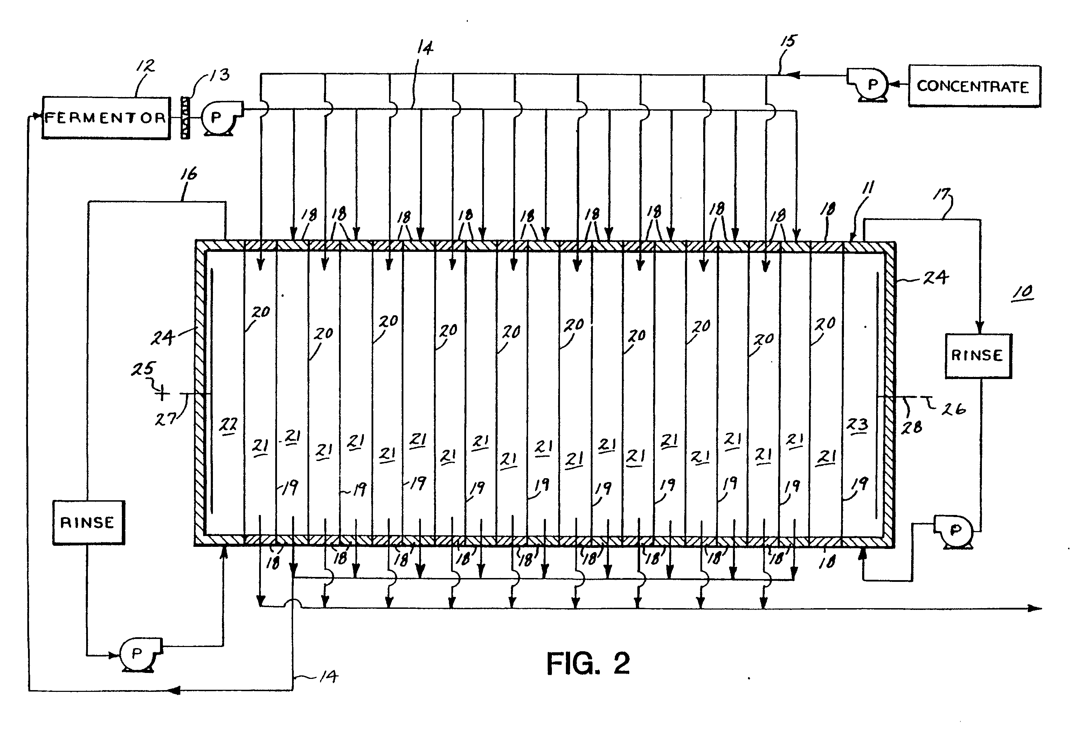

In Fig. 2 of the drawing, an electrodialysis apparatus 10 is shown which includes a cell stack 11, a fermentor 12, a screen 13, a feed solution injection, a recirculation and removal system 14, a concentrate recirculation and removal system 15, an anolyte rinse system 16 and a catholyte rinse system 17.

It will be appreciated that the apparatus illustrated in the drawing is for purposes of illustration and that modifications thereof can be made by those skilled in the art without departing from the scope of the present invention.

Cell stack 11 may be any known type of membrane assembly such as a plate and frame assembly wherein a plurality of suitably perforated flow distribution gaskets 18 support and seal the peripheries of a plurality of anion-permeable membranes 19 and cation-permeable membranes 20 in parallel spaced relation to form a series of parallel cells 21 and end cells 22 and 23. Each cell 21 is defined by a pair of membranes 19 and 20 and a gasket 18. The end cells 22 and 23 are respectively defined by a membrane 19 and a membrane 20 and end caps 24. Disposed within end cell 22 is a suitable anode 25 and a cathode 26 is disposed in the opposite end cell 23. Anode 25 and cathode 26 are connected respectively to the positive and negative terminals of a suitable power source (not shown) through leads 27 and 28. Cell stack 11 also includes suitable couplings (not shown) for admitting and removing liquids from each of cells 21. The components of the cell stack 11 can be held in an abutting relation by suitable clamps or tie rods (not shown).

The membranes 19 are anion-permeable and cation-impermeable and the membranes 20 are cation-permeable and anion-impermeable membranes. Suitable materials for use as membranes 19 and 20 include anion and cation exchange resins having active ion capture sites, respectively. The preferred membranes 19 are fabric reinforced microheterogeneous interpolymer membranes, such as the anion exchange membranes manufactured by Asahi Glass Co. SELEMION AMV membranes, while the preferred membranes 20 are the SELEMION type CMR cation exchange membranes available from the same source. The membranes are described in U.S. Patent No. 4,678,553 and others.

In the preferred practice of the invention, the whole broth is pumped from the fermentor 12 through the screen 13 which removes larger than cell size particles. The whole broth, minus those particles, is transported via system 14 to the cells 21 where it is subjected to an electrical current which causes the lactate ions to migrate through the permeable membrane 19 into a concentrated lactate salt solution which is circulating in the system 15 on the other side of the membrane 19. As seen in Fig. 1, the lactate salt enriched solution, also called the concentrate, is transmitted to a water-splitting electrodialysis apparatus as is or after further concentration to convert the lactate salt to lactic acid. The feed solution, minus the lactate salt, but including the viable cells is recirculated back to the fermentor. While the electrodialysis is going on the electrodes are rinsed with a suitable electrolyte solution, such as $Na_2SO_4$ or lactate salt in water, circulated by the rinse systems 16 and 17. The process can be operated on either a batch or continuous basis.

The present invention is further described and illustrated by the following experimental work:

Lactobacillus acidophilus ATCC 53681 was fermented on a medium consisting of 10% dextrose, 2% Corn Steep Liquor (CSL) dry basis 0.4% corn oil, unless otherwise noted. The media solution was placed in the appropriate fermentor and sterilized prior to inoculation. Five percent (v/v) inoculum, in the logarithmic growth phase was used.

Continuous fermentations were performed in a 1 l spinner flask which was properly sealed and sterilized prior to use. Peristaltic pumps were used to deliver fresh medium to and remove spent broth from the continuous fermentors. The pH was controlled at between 5.2 - 5.6 using concentrated ammonium hydroxide. The fermentation temperature was controlled at 40°C.

Cell recycle fermentations were conducted using a continuous culture fermentor with a filter. The fermentor broth was pumped across the surface of the filter and the cell-free permeate was collected in an effluent container and the cell-enriched retentate was returned to the fermentor. Temperature, media and pH were all as previously stated.

For testing the electrodialysis recovery process large fermentation broth volumes were prepared. An 80 l pilot plant fermentor and a 500 l fermentor were used to prepare large volumes of ammonium or sodium lactate fermentation broths. Media was prepared as described earlier. A 5% (v/v) inoculum was used. The pH was maintained at 5.2 - 5.6 by addition of ammonium hydroxide or sodium hydroxide on a demand basis. Agitation speed was 75 rpm.

The cells in the fermentation broth, when indicated, were removed by processing the broth through a ultra-filtration unit with a hollow fiber cartridge of 0.2 micron pore size.

Freshly prepared whole broth (containing viable cells) from the same fermentation was used for the whole broth experiments. The whole broth was screened through a 200 mesh wire screen to remove large particles before being charged to the electrodialysis system.

The product of the fermentation was then purified by first running it through a conventional electrodialysis unit equipped with membranes which were selective for anions and cations.

A two compartment bipolar membrane stack was used for processing lactate salts into lactic acid and the corresponding base. The stack designed for this process consisted of alternating cation permeable and bipolar membranes. Anode and cathode compartments are bound by a nafion membrane at each end of the membrane stack. The membrane stack contained the following:

8 cell pairs

-cation membrane

-bipolar membrane

effective area 102. 4cm$^2$

electrolyte (2.5 N NaOH)

The unit consists of three independent flow channels fed to the electrodialyzer stack. The three streams are:

1. Acid stream (initially the sodium lactate salt stream)
2. Base stream (becomes more concentrated as run proceeds)
3. Electrode rinse stream (2.5 N NaOH)

Before making actual runs, permissible current density (PCD) was determined to obtain a safe range for operating current density. For these runs, only two reservoirs were used. One reservoir supplied broth to both the diluting and concentrating compartments and then was pumped back to the reservoir. The second reservoir was for the electrolyte.

The unit was operated at low current and the voltage was recorded every 30 seconds for a period of 30 minutes to two hours. If the voltage remained constant over time, the procedure was repeated at a higher current. This procedure was continued until a point was reached when the voltage began to increase with time denoting the onset of polarization or fouling. The current density at that point was the PCD for that operating condition (salt concentration, pH, temperature, and linear velocity). The initial operating current density (12 Amps, 65 mA/CM$^2$) was chosen based on the PCD results.

The conventional and water-splitting electrodialysis systems were operated in a batch mode. Thus, the lactate broth was continually being demineralized (conventional ED) or converted to lactic acid and alkali (water-splitting ED). The conventional electrodialysis process continued until the solution was demineralized to a desired degree. The water-splitting ED process continued until the desired degree of acidity was obtained.

To run conventional ED the following procedure was done:

(i) The dilute reservoir was filled with sodium lactate broth;

(ii) The concentrate reservoir was filled with sodium lactate solution of concentration approximately equal to the feed broth;

(iii) The electrolyte compartment was filled with 1M sodium lactate or .4M sodium sulfate or another suitable salt.

(iv) Circulation of the fluids was started and the desired current density was applied to the membrane stack;

(v) During the run, data collected included: current, voltage drop across total unit, voltage drop across eight cell pairs, volume levels in the diluting and concentrating compartments, pH of the diluting and electrolyte compartments, conductivity of the diluting compartment, and temperature of the diluting compartment;

(vi) The unit was operated at a constant current until the resistance started to increase thus increasing the voltage drop across the unit (at that time, a maximum voltage was set and the current was allowed to decrease until the end of the run).

Water-splitting electrodialysis was done as follows:

(i) The acid reservoir was filled with sodium lactate concentrate or evaporated concentrate from conventional ED;

(ii) The base reservoir was filled with 1.5M sodium hydroxide. (When the base concentration reaches 2.5M, part of the base was removed and the remainder was diluted.)

(iii) The electrode rinse reservoir was filled with 2.5M sodium hydroxide;

(iv) Circulation of the solutions was started and the desired current was applied to the membrane stack;

(v) During the run the following data was collected: current, voltage across the unit, volume in the acid and base compartments, pH of the acid compartment, base normality, quantity of base removed, temperature of the acid compartment, and the acid and base compartment conductivities;

(vi) The unit was operated at a constant current until the voltage rises to 25 volts, then the voltage was maintained at 25 volts and the current was allowed to decrease until the end of the batch recovery.

After the water-splitting electrodialysis residual cations, anions and amino acids remain in the acid process stream. A strongly acidic cation exchanger, such as Amberlite IR-120 plus in the H$^+$ cycle (Rohm & Haas Co., Philadelphia, PA) was used to remove the cation impurities. A weakly basic anion exchanger, such as IRA-94 in free base cycle (Rohm & Haas Co., Philadelphia, PA) was used to remove the contaminant anions such as sulfate.

Two inch glass columns were used for ion exchange polishing of the lactic acid process stream. The columns were operated in the continuous flow mode. Absorption was conducted using volumetric flow rates of 5-10 bed volumes per hour. Regeneration of columns was accomplished using dilute (1-2M) sodium hydroxide or hydrochloric acid.

Examples 1-5

Continuous and Batch Fermentations

In continuous culture at pH 5.2 - 5.6 controlled with ammonium hydroxide, the volumetric productivity increased with increasing dilution rate to a maximum value of about 1.5 g lactate/l-hr at a dilution rate of 0.1/hr and did not change with increasing dilution rate up to 0.40/hr. The conversion efficiency (g lactic acid/g dextrose consumed x 100%) dropped from about 93% to 81% as the dilution rate was increased to 0.4/hr. This is not surprising since lactate production at high dilution rate becomes completely growth related. Thus, the amount of substrate converted to cells increases, and the amount of substrate converted into product lactate decreases.

## Examples 6-14

The effect of pH on continuous cultures was also evaluated. Cell growth appeared nearly independent of pH in all cases when the pH was above 5.3. At lactate concentrations below 20 g/l and dilution rates above 0.1/hr, growth was independent of pH when the pH was above 4.8. This indicates that it would be possible to run a multistage continuous fermentation in which the pH ranges from low (e.g. 4.8) in the first stage to moderately high (e.g. 5.3 to 5.6) in the final stage and thus reduce the chances of contamination without inhibiting growth of the production culture.

## Examples 15 and 16

In a commercial lactate fermentation process, high productivity must be maintained in a product stream that has high lactate and low substrate concentrations. A good candidate organism for such a process must be able to maintain viability in the presence of low substrate and high product concentrations. This is important since productivity is dependent upon the concentration of viable cells in the fermentor. This concentration can be increased by retaining a high percentage of the cells in the fermentor (e.g. through cell recycle or immobilization). However, if the cells lose viability under the conditions of the final product stream, they will be inactive and of no value in either a recycle or immobilization process.

Viable cell, lactic acid, and residual carbohydrate (dextrose) concentrations were measured during a pH-controlled (5.2 - 5.6) batch fermentation using 2% CSL medium and strain L. acidophilus ATCC 53861. The viability of the culture remained high ($5 \times 10^{10}$ viable cells/ml) despite the depletion of all carbohydrate. This indicated that the cells would be able to retain viability in a continuous fermentation with recycle and contribute to an increased volumetric productivity.

Continuous cultures of the strain were run with 100% cell recycle to determine the maximum achievable volumetric productivity. The results are summarized in Table 3. Volumetric productivities of 7.5 g lactate/l-hr were achieved using 2% CSL medium. It was demonstrated that 95% conversion efficiency was feasible.

The fermentations have shown that using the organism Lactobacillus acidophilus ATCC 53861 and carefully selected operating conditions a high volumetric productivity, using a low cost media, can be achieved. Using cell recycle and pH control at 5.2 - 5.6 a productivity of 7.5 g lactate/l hr was achieved. The high productivity on a low cost media is necessary for an economic process to process to produce lactic acid.

## Examples 17 and 18

Batch fermentations to produce sodium and ammonium lactate broths were conducted. The lactate containing broths were used for electrodialysis product recovery trials. These fermentations show that 80 and 500 l fermentations gave the same productivity and yield as small bench scale 2 l fermentations.

The 65 l fermentation yielded a sodium lactate broth containing 86 g/l lactate. The 150 l fermentation yielded an ammonium lactate broth containing 81 g/l lactate. A portion of these fermentation broths were clarified using an Amicon DC-30 hollow fiber cartridge with a pore size of .2 um to remove the cells. The remaining whole broth was run through the electrodialysis recovery process directly after completion of the fermentation.


## Examples 19 and 20


Two electrodialysis recovery experiments were done with whole culture lactate broth without cell removal (containing 2.5 - 3 x $10^{10}$ cell/ml). The first was a batch run to determine feasibility which was followed by a second batch run at a slightly lower temperature, 45° C, to maintain viability of the cells. The lactate concentration initially was about 77 g/l. The final purified and concentrated lactate concentration was about 175 g/l. Removal of 80% of the lactate was achieved with a power requirement of .08 to .11 kwhr/lb lactate. The current efficiency in both runs was about 90%. Fouling due to the presence of whole cells was not detected in either run.

During the whole broth electrodialysis run, samples of the whole broth were assayed for viable cells (by plate count on MRS. agar media). The data showed that the cells remain predominantly viable. It was difficult to further quantify cell viability with much precision because of the inherent inaccuracies in the plate count assay.

The electrodialyzed broth from Example 20 was further fermented with additional dextrose, corn oil (0.2%), and different concentrations of corn steep liquor (F2-1.4%, F3-2.4%, F4-0%). The fermentation volume was made up to the original broth volume (0.7 liter to 1 liter). The viable cell count for F2 and F3 at the beginning of recycle fermentation was comparable to the cell count in the electrodialyzer feed broth. The recycle fermentations F2 and F3 proceeded rapidly producing 77 and 85 g/l lactate respectively in 24 hours with productivities of 2.2 and 2.5 g/l-hr, respectively. These were comparable to that obtained in typical batch fermentations, 2-2.5 g/l-hr. Recycle fermentation F4 (0% CSL) was slow and required 100 hours to consume the dextrose and produce 90.4 g/l lactate. This result showed that viable cells from the electrodialysis of whole broth can be recycled and used to ferment more carbohydrates to lactate without losing productivity and the ability to produce high lactate concentration.


## Examples 21, 22 and 23


The results of electrodialysis recovery of lactate using clarified ammonium lactate broth are shown in Table 1. A final lactate concentration of 200 g/l was achieved from a feed concentration of 80 g/l lactate. The current efficiency for the runs was about 92%. The power consumption was .08 to .12 kwhr/lb. lactate recovered.

Table 1

| Electrodialysis of Clarified Ammonium Lactate Broth from L. acidophilus Fermentation | | | |
|---|---|---|---|
| | Ex. 21 | Ex. 22 | Ex. 23 |
| Broth/Dilute Concentrations | | | |
| Initial Lactate (g/l) | 81 | 75 | 82 |
| Final Lactate (g/l) | 9 | 9 | 19 |
| Product/Concentrate Concentrations: | | | |
| Initial Lactate (g/l) | 52 | 72 | 62 |
| Final Lactate (g/l) | 203 | 196 | 191 |
| Lactate Removal (%) | 92 | 92 | 82 |
| Length of Run (minutes) | 150 | 140 | 120 |
| Temperature ($^\circ$C) | 50 | 50 | 50 |
| Current Efficiency (%) | 92 | 88 | 96 |
| Initial Current Density (mamp/cm$^2$) | 67 | 67 | 67 |
| Electricity Requirement (kwhr/lb) | .08 | .08 | .12 |

## Example 24

Table 2 summarizes the results of electrodialysis recovery using sodium lactate containing fermentation broth. Ninety-four percent of the lactate was recovered. The initial lactate concentration in the broth was 84 g/l. The product lactate concentration was 205 g/l. The current efficiency was 96% and the power requirement was .18 kwhr/lb lactate recovered. The power consumption for this run was higher than other runs partly because a higher percentage of the lactate was recovered. The low concentration and conductivity of the dilute stream at the end of the batch run increases the power consumption.

Table 2

| Electrodialysis of Clarified Sodium Lactate Broth From L . acidophilus Fermentation | |
|---|---|
| | Ex. 24 |
| Broth/Dilute Concentrations: | |
| Initial Lactate (g/l) | 84.1 |
| Final Lactate (g/l) | 5.7 |
| Product/Concentrate Concentrations: | |
| Initial Lactate (g/l) | 51.1 |
| Final Lactate (g/l) | 204.6 |
| Lactate Removal (%) | 94.4 |
| Length of Run (minutes) | 135 |
| Temperature ($^\circ$C) | 47 |
| Current Efficiency (%) | 96 |
| Initial Current Density (mamp/cm$^2$) 67 | |
| Electricity Requirement (Kwhr/lb) | .18 |

The significance of electrodialysis results using whole broth or clarified broth was that the presence of whole cells in the broth does not cause any additional power consumption or reduction of current efficiency. This means that with proper choice of operating conditions the presence of whole cells in the dilute ED compartment does not negatively affect ED operation. It was also demonstrated that the electrodialysis operation had no effect on the viability of the whole cells. Therefore, conventional electrodialysis can be used to recover, purify and concentrate the lactate product and to recycle whole cells back to the fermentor.

## Examples 25, 26 and 27

The concentrated lactate salt needs additional processing to remove the cation and obtain the desired lactic acid product. A water-splitting electrodialysis membrane stack (Allied/Aquatech) was used to remove 85-99 percent of the sodium cations from the lactate salt stream. The products were alkali for recycle to the fermentor for pH control and a lactic acid stream.

The lactate salt after conventional electrodialysis was about 20 weight percent lactate. There are two good reasons to concentrate the lactate salt prior to doing the water-splitting ED. They are:

(i) Typical specialty and commodity chemical uses of lactic acid require at least 50% lactic acid by weight;

(ii) Power consumption is reduced when the conductivity of the solution being electrodialyzed is increased. A low residual sodium contamination is easier to achieve using the concentrated lactate salt as a feedstock.

Because of these reasons the lactate salt was concentrated prior to water-splitting ED for part of the runs.

The sodium cations were removed from the sodium lactate salt using a water-splitting ED membrane stack. A 2.0 N sodium hydroxide solution and a lactic acid stream (same concentration as the feed) were the resulting products. Results from ED runs using 2.4 and 5 normal lactate salt streams are summarized in Table 3. A comparison of the runs show that the power consumption is lowest when a lesser percentage of the sodium is removed. In the extreme case, comparing runs Examples 26 and 27, power consumption was greatly increased to remove a small portion of the initial sodium present. The power consumption per pound of lactic acid produced was between 0.25 and 0.45 Kwhr.

Table 3

| Water-Splitting Electrodialysis of Sodium Lactate Concentrate From Conventional Electrodialysis | | | |
|---|---|---|---|
| | Ex. 25 | Ex. 26 | Ex. 27 |
| Sodium Concentrations (g/l) | | | |
| Initial | 44.6 | 93.8 | 112.7 |
| Final | 5.8 | 0.7 | 0.3 |
| Salt/Acid Stream Concentrations: | | | |
| Initial Lactate (g/l) | 175 | 415 | 441 |
| Final Lactate (g/l) | 198 | 450 | 470 |
| Sodium Removal (%) | 87 | 99 | 99.5 |
| Length of Run (minutes) | 115 | 225 | 270 |
| Temperature ($^{\circ}$C) | 45 | 45 | 45 |
| Current Efficiency (%) | 84 | 78.5 | 68 |
| Initial Current Density (mamp/cm$^2$) | 127 | 127 | 127 |
| Electricity Requirement (kwhr/lb) | .27 | .33 | .45 |

Recovery of lactic acid from fermentation broth has been accomplished using two electrodialysis membrane stacks and approximately 1/2 kwhr of electricity per pound of lactic acid. This recovery, concentration and purification process forms the heart of an economical process for production of lactic acid.

## Example 28

Results from the water-splitting electrodialysis runs indicated that residual sodium ions needed to be removed from the lactic acid stream. Sulfate and other anions as well as proteins or amino acids were also present as contaminants at this point. Table 4 shows the composition of the process stream at various stages of the process.

Table 4

| Lactic Acid Ion Exchange Polish Purification Summary | | | |
|---|---|---|---|
| | Stream Compositions (Weight %, dry basis) | | |
| | Before Ion Exchange | After Cation Exchange | After Anion Exchange |
| Lactate | 94.7 | 98.8 | 99.9 |
| Protein (Kjeldahl N x 6.25) | 1.7 | 0.8 | - |
| Sodium | 3.2 | - | - |
| Sulfate | 0.4 | 0.4 | - |

Strongly acidic cation exchange resin Amberlite IR-120 plus (Rohm & Haas Co.) in the H+ cycle was used in a continuous flow column to remove the residual sodium ($Na^+$) and other cations. The lactic acid was not exchanged into this resin. Some of the nitrogenous impurities (amino acids and proteins) were also removed along with the sodium ions (Table 4).

The product stream from the cation exchange purification step still contained anionic impurities such as sulfate ($SO_4^=$) and nitrogenous impurities (proteins and amino acids). Removal of the anionic impurities from lactic acid which contains the lactate anion was a difficult task because the ion exchange sites could be saturated with the lactate anion and not remove the impurity anions. Certain weakly basic anion exchange resins, among them Amberlite IRA-94 (Rohm & Haas Co., Philadelphia, PA), in the free base cycle were found to be suitable. The resin was used in a continuous flow column. The data (Table 10) show that the $SO_4^=$ anion impurity was completely removed whereas the lactate in lactic acid was not ion exchanged. Fortuitously the residual nitrogenous impurities were removed and a product lactic acid was greater than 99% pure. This product was also fairly concentrated and contained 440 g/l lactic acid.

The results in Table 4 show that purification of the lactic acid can be accomplished by using specific ion exchangers in the proper sequence. The cation exchanger must be used first followed by the anion exchanger to successfully remove the contaminants. The anion exchanger must be especially carefully chosen because the lactate is also an anion.

Thus, by the careful selection of processing steps a product containing only 0.1% of protein impurities and less than 5 ppm sulfate and sodium ions can be produced by the described fermentation and purification process.

It will be apparent to those skilled in the art that the combination of a robust strain of L. acidophilus and the use of fabric reinforced, microheterogeneous interpolymer membranes permits simultaneous electrodialysis and cell recycle from whole broth to be achieved without loss of efficiency either in the fermentor or the electrodialysis separator. The mature cells remain viable and are recycled to the fermentor where fermentation continues with addition of carbohydrates and nutrients, and the purified and concentrated lactate from the electrodialyzer can be used to feed the water-splitting electrodialysis system, such as the "AQUATECH" system, where the lactate salt is converted to lactic acid and the corresponding base. The base obtained can be recycled to the fermentor for neutralization, and purified concentrated lactic acid further purified with ion exchangers for use as a specialty chemical or a commodity chemical intermediate.

Following the purification and recovery of the lactate salt by conventional electrodialysis the salt can be concentrated further by evaporation to 45 to 50% lactate salt before water-splitting electrodialysis is done.

It is extremely important that the lactate salt stream from the fermentation be first treated by conventional electrodialysis to recover the lactate salt, recycle viable cells and remove the nitrogenous impurities; that it next be treated with water-splitting electrodialysis to convert the lactate salt to lactic acid and that the resulting lactic acid stream be treated first with a strongly acidic ion exchanger and then a

EP 0 393 818 A1

weakly basic ion exchanger to obtain the desired product.

**Claims**

1. A process of producing and purifying lactic acid which comprises:

(a) growing an organism capable of producing lactate salt in a fermentor on a substrate containing carbohydrate and other nutrients until most of the carbohydrate in the fermentation broth is converted to a lactate salt;

(b) subjecting the lactate salt-containing broth first to conventional electrodialysis to preferentially recover and concentrate the lactate salt in an aqueous stream and to remove nitrogenous impurities;

(c) subjecting the aqueous stream containing the concentrated lactate salt to water-splitting electrodialysis to convert the lactate salt to corresponding base and a lactic acid stream;

(d) treating the lactic acid stream with a strongly acidic ion exchanger in the acid form to remove any sodium or other cations from the stream without removing the lactic acid; and,

(e) treating the thus treated lactic acid with a weakly basic ion exchanger in the free base form to remove any sulfuric acid or sulfate and other impurities from the stream without removing lactic acid and to obtain a purified lactic acid product.

2. A process according to claim 1, in which the organism is an organism having the identifying characteristic of L. acidophilus (ATCC 53861).

3. A process according to claim 1 or 2, in which any viable cells in the broth from which lactate salt has been removed are recycled to the fermentor.

4. A process according to any preceding claim, wherein the base obtained by the water-splitting electrodialysis is returned to the fermentor.

5. A process according to claim 1, in which the lactate salt in the aqueous stream is concentrated by evaporation prior to the water-splitting electrodialysis to convert the lactate salt to base and a lactic acid stream.

6. A lactic acid product prepared by the process of any prededing claim, which contains lactic acid, less than 1% nitrogenous impurities and less than 10 ppm of sulfate ions.

7. A process for producing a highly purified lactic acid product which comprises:

(a) growing an organism having the identifying characteristics of L. acidophilus (ATCC 53861) in a fermentor on a substrate containing carbohydrates, corn steep liquor and corn oil at a pH of about 4.8 to about 5.7 until most of the carbohydrate in the fermentation broth is converted to a lactate salt;

(b) treating the fermentation broth by electrodialysis to remove nitrogenous impurities and isolate the lactate salt in a aqueous stream;

(c) treating the aqueous stream of water-splitting electrodialysis to form a base and lactic acid stream;

(d) treating the lactic acid stream with a strongly acidic ion exchanger in the acid form to remove any sodium or other cations from the stream without removing lactic acid; and

(e) treating the resulting lactic acid stream with a weakly basic ion exchanger in the free base form to remove any sulfuric acid or sulfate impurities and other impurities without removing lactic acid and to obtain a lactic acid product containing lactic acid, and less than 1% nitrogenous impurities.

8. A process according to claim 7, in which the corn oil is present in at least about 0.1% by weight.

9. A process according to claim 7 or 8, in which the lactate salt in the aqueous stream is concentrated by evaporation prior to the water-splitting electrodialysis to convert the lactate salt to base and a lactic acid stream.

10. A lactic acid product obtained by the process according to any one of claims 7 to 9, which contains less than 1% of nitrogenous impurities.

11. A method of purifying a lactic acid solution containing lactic, sulfuric acid, sulfate ions, sodium ions and other cationic impurities which comprises:

(a) treating the solution first with a strongly acidic ion exchanger in the acid form which removes the sodium or other cations without removing lactic acid; and,

(b) treating the thus obtained solution with a weakly basic ion exchanger in the free base form which removes the sulfate ions and sulfuric acid and other impurities without removing the lactic acid.

11

FIG. 1

CELL RECYCLE

PURGE STREAM

CARBOHYDRATES →

NUTRIENTS →

FERMENTATION —BROTH→ ELECTRODIALYSIS —Lactate→ WATER-SPLITTING ELECTRODIALYSIS —Lactic Acid→ ION EXCHANGERS —→ Purified Lactic Acid

BASE RECYCLE

EP 0 393 818 A1

FIG. 2

EP 0 393 818 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 230 021 (HÜLS AG) <br> * Claims; examples * <br> --- | 1 | C 12 P 7/56 |
| Y | EP-A-0 265 409 (SYNFINE-OLEOFINE) <br> * Claims * <br> ----- | 1 | |

|  |  |
|---|---|
|  | TECHNICAL FIELDS SEARCHED (Int. Cl.5) |
|  | C 12 P <br> C 12 M <br> A 23 C |

The present search report has been drawn up for all claims

| Place of search | Date of completion of the search | Examiner |
|---|---|---|
| THE HAGUE | 16-07-1990 | DELANGHE L.L.M. |